# EUROPEAN PATENT APPLICATION

(11) **EP 2 755 012 A1**
(43) Date of publication of application: **16.07.2014**
(21) Application number: 12829352.9
(22) Date of filing: 03.09.2012
(51) Int. Cl.: G01N 1/00, G01N 1/22, G01N 21/03, G01N 21/15, G01N 21/35, G01N 31/00

(54) **GAS ANALYSIS DEVICE AND CONTAMINATION DETECTION METHOD USED IN SAME**

(30) Priority: 08.09.2011 JP 2011196361
(71) Applicant: HORIBA, Ltd., Minami-ku Kyoto-shi Kyoto 601-8510 (JP)
(72) Inventor: ITAYA, Takahiro, Kyoto-shi Kyoto 601-8510 (JP); NAKATANI, Shigeru, Kyoto-shi Kyoto 601-8510 (JP)
(74) Representative: Müller - Hoffmann & Partner
(86) International application number: PCT/JP2012/072379
(87) International publication number: WO 2013/035675

(57) **Abstract**

In order to make it possible to sensitively detect adsorptive gas even in the presence of a small amount of contamination that significantly influences adsorptive gas measurement, a gas injecting mechanism 3 that injects adsorptive injection gas into a flow path 11 through which sample gas is flowed; a gas measuring mechanism 21 that can measure a value related to quantity of the adsorptive gas flowing through the flow path 11; and a contamination determining part 41 that determines contamination in the flow path on the basis of a value related to a measurement response speed of the gas measuring mechanism 21 to the adsorptive gas are provided.

## Description

### Technical Field

The present invention relates to a gas analysis device that can detect whether or not contamination occurs in a flow path through which gas as a measuring target flows.

### Background Art

For example, a component such as NO_{X} in exhaust gas emitted from an internal combustion engine of an automobile is measured. In recent years, in this sort of exhaust gas analysis device, analysis of adsorptive gas such as NH₃ that is a component other than NO_{X} becomes increasingly important.

Specific examples of measuring gas having adsorptivity, such as NH₃, include a scene of research and development of a urea SCR (Selective Catalytic Reduction) system that can efficiently drive a diesel engine and also suppress a production amount of NO_{X}, and other scenes. To specifically describe the urea SCR system, the urea SCR system is one that is configured to, by spraying urea into high-temperature exhaust gas emitted from a diesel engine, and as a reducing agent, supplying NH₃ produced by pyrolysis of the urea to an SCR catalyst, reduce NO_{X} in the exhaust gas to change the NO_{X} to harmless N₂ and H₂O.

In the case where an excessive amount of urea is supplied in such a urea SCR system, NH₃ is contained in the exhaust gas to give rise to a bad odor, or failure to meet environmental standards. For this reason, in order to know whether or not an adequate amount of urea can be supplied in various driving conditions, NH₃ in the exhaust gas is measured.

Meanwhile, differently from gas as a conventional measuring target, such as NO_{X}, in the case of the gas having adsorptivity, such as NH₃, NH₃ is adsorbed on an inner wall or the like of a pipe before arriving at a gas analyzing mechanism capable of measuring an amount of NH₃, and therefore it is difficult to measure an accurate value in real time. In particular, in the case where due to soot and the like contained in exhaust gas, contamination is attached on the inner wall or the like of the pipe, a response speed of the gas measuring mechanism to such adsorptive gas is more deteriorated.

From the viewpoint of preventing a reduction in response speed, even as a conventional exhaust gas analysis device, for example, as disclosed in Patent Literature 1, there is one that is adapted to be provided with: a cell in which nonadsorptive gas such as NO is measured; and a mechanism that detects contamination in a flow path to the cell, and when contamination is detected, appropriately perform cleaning or the like.

Specifically, this exhaust gas analysis device is one that is intended to improve a response speed to the nonadsorptive gas such as NO_{X}, and therefore adapted to sense contamination on the basis that a large amount of contamination is accumulated to narrow the flow path, and an inflow flow rate of sample gas to the cell does not reach a specified value.

However, in the case of measuring gas having adsorptivity, even in the case where a small amount of contamination is attached inside a pipe forming the flow path to change an area of a surface in contact with gas in the pipe, an adsorption amount is significantly changed to give rise to a reduction in response speed. That is, it is necessary to detect contamination in an early stage more sensitively than the conventional contamination detecting mechanism, and clean an inner surface.

### Citation List

### Patent Literature

Patent Literature 1: JP-A2002-310910

### Summary of Invention

### Technical Problem

The present invention is made in consideration of the problem as described above, and intended to provide a gas analysis device provided with a mechanism that can sensitively detect even a small amount of contamination that significantly influences measurement of gas having adsorptivity, and also a contamination detection method used for the gas analysis device. Solution to Problem

That is, the gas analysis device of the present invention is provided with: a gas injecting mechanism that injects adsorptive injection gas into a flow path through which sample gas is flowed; a gas measuring mechanism that can measure a value related to quantity of the adsorptive injection gas flowing through the flow path; and a contamination determining part that determines contamination in the flow path on the basis of a value related to a measurement response speed of the gas measuring mechanism to the adsorptive injection gas.

Also, the contamination detection method of the present invention is a contamination detection method for a flow path through which sample gas is flowed in a gas analysis device, and provided with: a gas injecting step of injecting adsorptive injection gas into the flow path; a gas measuring step of measuring a value related to quantity of the adsorptive injection gas flowing through the flow path; and a contamination determining step of determining contamination in the flow path on the basis of a value related to a measurement response speed to the adsorptive injection gas in the gas measuring step. Note that "determining contamination" is a concept including not only determining whether the contamination such as soot is present but also determining a level of the contamination. Also, the value related to the measurement response speed to the adsorptive injection gas is a concept including, for example, not only a response speed or a response time, but calculating a response speed on the basis of a concentration value of the injection gas, which is measured within a predetermined period of time.

If so, the contamination determining part is configured to determine contamination on the basis of the value related to the measurement response speed of the gas measuring mechanism to the adsorptive injection gas that is flowed through the flow path, and the measurement response speed to the adsorptive injection gas is changed even in the case where a small amount of contamination is present, so that the presence or absence of contamination can be sensed more sensitively than before.

Accordingly, the flow path can be cleaned at an appropriate frequency even in the presence of a small amount of contamination in adsorptive gas measurement that is easily influenced by contamination, and therefore, for example, within a predetermined reference range of measurement response speed, gas having adsorptivity can be measured.

Specific aspects that make it possible for the contamination determining part to sense even a small amount of contamination to, in particular, favorably keep a response speed in adsorptive gas measurement include an aspect in which: the flow path is formed in a gas pipe through which the sample gas is flowed, and in a sampling pipe for sampling part of the sample gas; and the gas measuring mechanism is provided in the sampling pipe, and the contamination determining part is configured to determine contamination on an inner surface of the sampling pipe on the basis of the value related to the measurement response speed.

In order to make it possible to sense the presence or absence of contamination throughout a measurement system to favorably keep a response speed for a long period of time in adsorptive gas measurement, it is only necessary that the gas injecting mechanism is configured to inject the adsorptive injection gas into the gas pipe.

In order to make it possible to accurately detect even a small amount of contamination in spite of a simple configuration, it is only necessary that the contamination determining part is configured to determine that, in the case where a response time necessary for at least a predetermined amount of adsorptive injection gas to be measured by the gas measuring mechanism after the adsorptive injection gas has been injected by the gas injecting mechanism is equal to or more than a predetermined time, the contamination is present in the flow path.

### Advantageous Effects of Invention

As described, the present invention is configured to sense the presence of absence of contamination with use of a reduction in response speed, which is caused by the adsorption of the adsorptive injection gas on an inner surface, and can therefore accurately detect even a small amount of contamination. Accordingly, the flow path can be appropriately cleaned to favorably keep a response speed in adsorptive gas measurement that is influenced even by a small amount of contamination.

### Brief Description of Drawings

[Fig. 1]

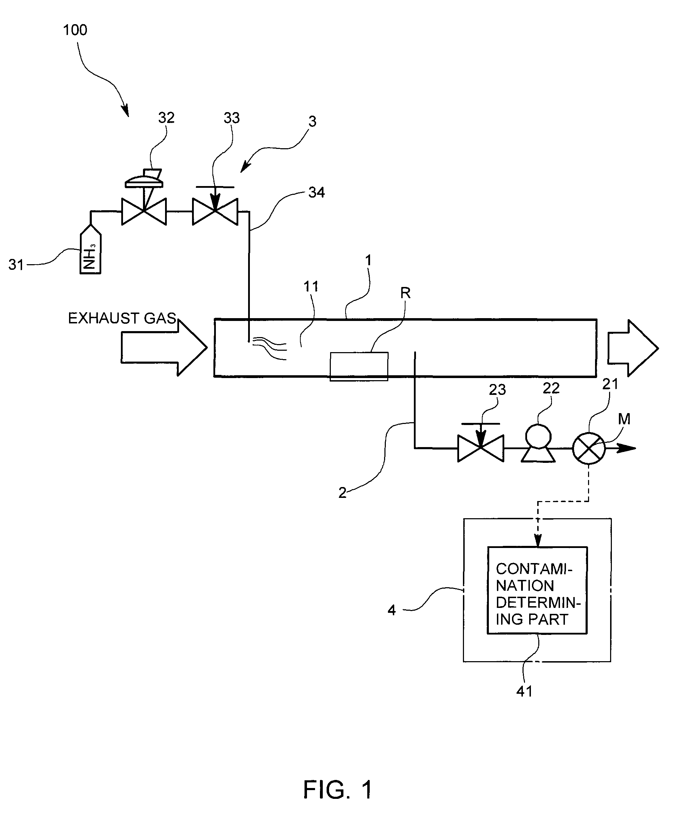

   Fig. 1 is a schematic diagram illustrating a configuration of an exhaust gas analysis device according to one embodiment of the present invention.
[Fig. 2]

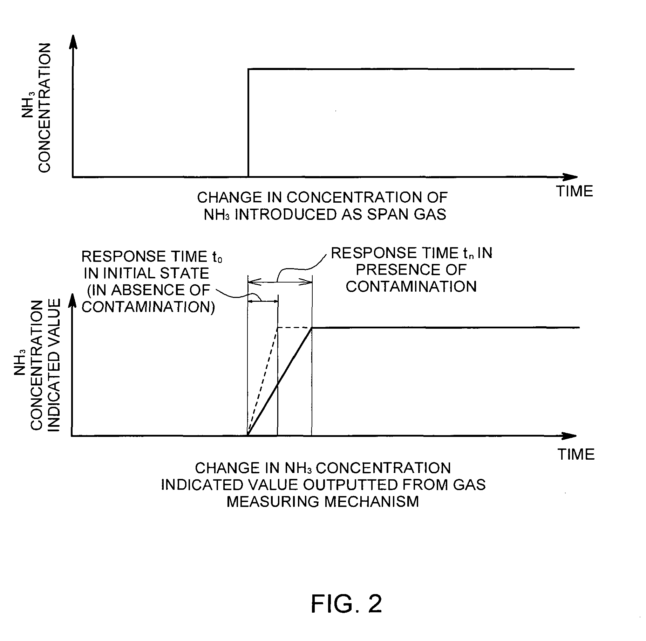

   Fig. 2 is a schematic graph illustrating a concentration change and a response speed at the time of determining contamination in the same embodiment.
[Fig. 3]

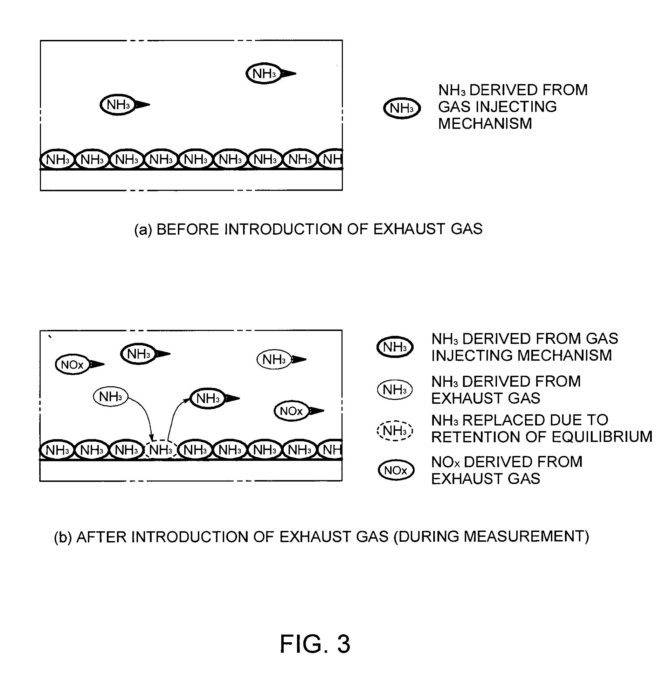

   Fig. 3 is a schematic diagram that enlarges a region indicated by an imaginary line in the schematic diagram of Fig. 1 and illustrates adsorption and peeling states of NH₃ on an inner surface.
[Fig. 4]

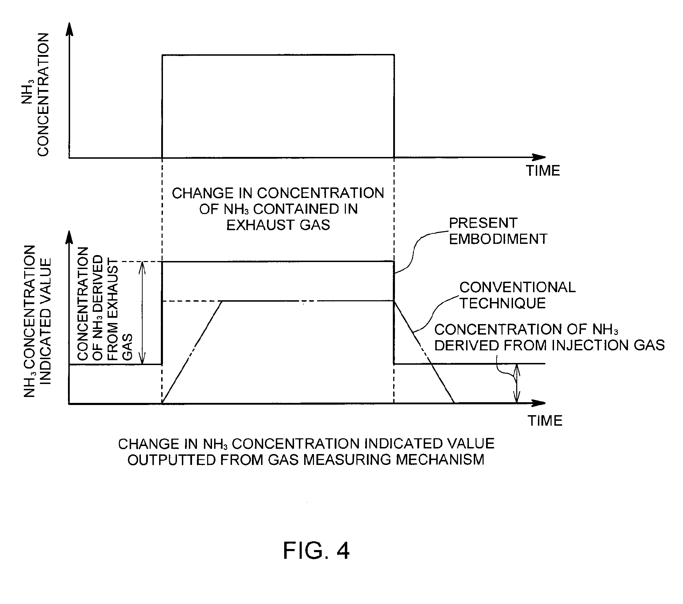

   Fig. 4 is a graph illustrating a difference in response at the time of measuring NH₃ concentration between the same embodiment and a conventional technique.
[Fig. 5]

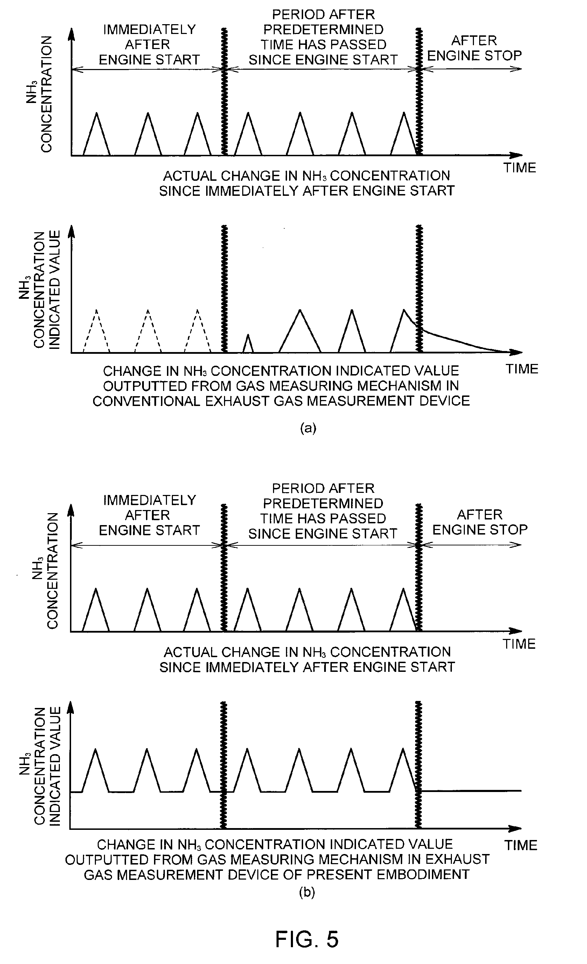

   Fig. 5 is a graph illustrating a difference in response at the time of measuring minute NH₃ concentration between the same embodiment and a conventional technique.
[Fig. 6]

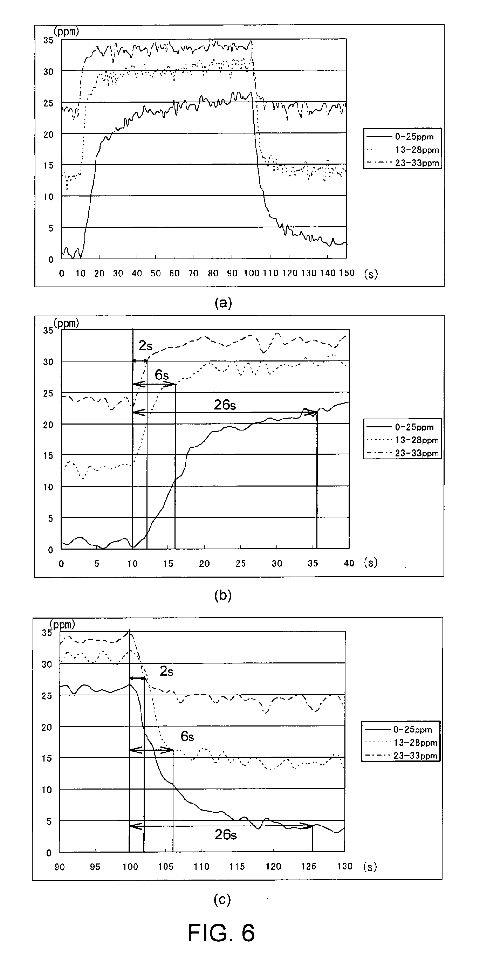

   Fig. 6 is a graph illustrating results of verifying a relationship among an injection gas amount, a rise time, and a fall time in the same embodiment. Reference Signs List

100: Exhaust gas analysis device (gas analysis device)
11: Flow path
21: Gas measuring mechanism
3: Gas injecting mechanism

### Description of Embodiments

One embodiment of the present invention is described with reference to drawings.

A gas analysis device of the present embodiment is a so-called exhaust gas analysis device 100, and is one that is used to measure the concentration of NH₃ contained in exhaust gas emitted from a diesel engine mounted with a urea SCR system.

More specifically, as illustrated in Fig. 1, the gas analysis device 100 is one that is provided with: a gas pipe 1 through which the exhaust gas as sample gas flows; a sampling pipe 2 for sampling part of the exhaust gas from inside the gas pipe 1; a gas measuring mechanism 21 that has a measuring point M in the sampling pipe 2 to measure the concentration of the NH₃ contained in the exhaust gas; a gas injecting mechanism 3 that injects gas having the same component as a measuring target gas having absorptivity in the gas pipe 1; and a control mechanism 4 that controls the respective parts. In other words, the gas pipe 1 and the sampling pipe 2 form a flow path 11 through which the exhaust gas flows. In addition, a region R surrounded by an imaginary line in Fig. 1 is one that indicates an after-mentioned part enlarged in Fig. 3.

The respective parts are described.

The gas pipe 1 is a substantially cylindrically shaped, for example, stainless steel pipe attached to an unillustrated muffler of an automobile, and an inner surface thereof in contact with the exhaust gas is surface-treated such as being electrolytically polished so as to prevent NO_{X}, soot, and the like from being easily attached. Also, the gas pipe 1 is adapted such that most of the exhaust gas introduced into the gas pipe 1 is directly led outside from an opening on a downstream side.

The sampling pipe 2 is a substantially thin cylindrically shaped stainless steel pipe bent in an L-shape, and one end thereof is adapted to be radially thrust to a central part of the gas pipe 1 and also opened inside the gas pipe 1 to be able to sample the part of the exhaust gas. The sampling pipe 2 is, sequentially from an upstream side, provided with an on/off valve 23, a suction pump 22, and the gas measuring mechanism 21. In the case of measuring the concentration of NH₃ gas in the exhaust gas, the on/off valve is opened, and also the exhaust gas is sucked by the suction pump 22 so as to flow into the sampling pipe 2 at a predetermined flow rate. In addition, an inner surface of the sampling pipe 2 is also surface-treated by electrolytic polishing or the like as with the gas pipe 1.

The gas measuring mechanism 21 is one that can simultaneously measure concentrations of various components such as NO_{X}, CO, CO₂, and carbon hydrides in addition to NH₃ contained in the exhaust gas by FTIR (Fourier Transform Infrared Spectroscopy), and configured to update and output the concentrations of the respective components measured at cyclic intervals of, for example, 1 second. That is, concentration indicated values of the various components contained in the exhaust gas can be updated in substantially real time. In addition, the measuring point M of the gas measuring mechanism 21 in the present embodiment corresponds to a place where the gas measuring mechanism 21 is provided in the sampling pipe 2.

The gas injecting mechanism 3 is one that, as injection gas, injects NH₃ having adsorptivity among the measuring target components into the gas pipe 1, and is provided with: a gas injecting pipe 34 of which one end is connected to an injection gas source 31 where NH₃ are accumulated, and the other end is opened in the gas pipe 1; an on/off valve 33 that is provided in the gas pipe 1; and a flow rate control valve 32. The gas injecting mechanism 3 is adapted to keep supplying a predetermined amount of NH₃ into the gas pipe 1 at least while the concentration of NH₃ in the exhaust gas is being measured by the gas measuring mechanism 21. Further, the gas injecting mechanism 3 is also used for introducing zero gas and span gas for NH₃ to thereby perform calibration before measurement.

To describe in detail a position where the gas injecting pipe 34 is opened in the gas pipe 1, the position where the injection gas is introduced is set upstream of the measuring point M of the gas measuring mechanism 21. More specifically, the position is located upstream of a place where the sampling pipe 2 is opened in the gas pipe 1, and the other end of the gas injection pipe 34 is opened near an opening on a side where the gas pipe 1 is attached to the muffler. That is, the present embodiment is configured such that, substantially throughout the flow path 11 from the side where the exhaust gas is introduced to the measuring point M, the NH₃ gas can be dispersed by the gas injecting mechanism 3. Further, in other words, in the pipe located upstream of the measuring point M, the injecting position for the injection gas is set such that an area of an inner surface in contact with both of NH₃ in the exhaust gas, which is the measuring target gas, and the injection gas is larger than an area of an inner surface in contact with only NH₃ in the exhaust gas. For example, the injecting position is set such that even in the case where adsorption takes place on the inner surface in contact with only the NH₃ in the exhaust gas, only an amount equal to or less than a measurement limit of the gas measuring mechanism 21 is adsorbed.

The control mechanism 4 is a so-called computer provided with an input/output interface, memory, CPU, A/D and D/A converters, and the like, and configured to execute a program stored in the memory to thereby perform control or the like of various valves, and fulfill a function as at least a contamination determining part 41.

The contamination determining part 41 is configured to, on the basis of a value related to a measurement response speed of the gas measuring mechanism 21 to the adsorptive gas, determine the presence or absence of contamination in the flow path 11. Specifically, the contamination determining part 41 is one that determines that in the case where the adsorptive gas of which concentration or a flow rate is known is introduced into the gas pipe 1 and the sampling pipe 2 by the gas injecting mechanism 3, and the value related to the measurement response speed calculated from a measured value outputted from the gas measuring mechanism 21 at the time is lower than a predetermined specified value, at least an allowable amount of contamination such as soot is attached on the inner surface in contact with the flow path 11 through which the exhaust gas as the sample gas flows.

In the present embodiment, the contamination determining part 41 determines the contamination on the basis of a response time necessary to transit from a state where no measurement is made as the value related to the measurement response speed to a state where the value related to the measurement response speed is stabilized at a predetermined value, and is configured to determine that, in the case where the response time exceeds a predetermined specified time, at least the allowable amount of contamination is attached on the inner surface of the gas pipe 1 or sampling pipe 2.

A series of operations at the time of measuring the NH₃ gas, and the like, in the exhaust gas analysis device 100 configured as described are described.

First, operation performed by the contamination determining part 41 to sense contamination in the flow path 11 through which the exhaust gas flows is described.

Before the gas measuring mechanism 21 starts to measure the NH₃ in the exhaust gas, an output value from the gas measuring mechanism 21 is calibrated with the zero gas and span gas injected by the gas injecting mechanism 3. In addition, the present embodiment is adapted such that while contamination is being sensed, the sample gas such as the exhaust gas is not introduced into the gas pipe 1.

The contamination determining part 41 measures a response time tn that is a time necessary for the concentration of the NH₃ gas, which is measured by the gas measuring mechanism 21, to be stabilized after the span gas of which NH₃ concentration is set to concentration near a full scale of the gas measuring mechanism 21 has been injected into the gas pipe 1.

As illustrated here in Fig. 2, it turns out that the response time tₙ in the presence of contamination is longer than a response time to in an initial state where no contamination is present. This is because in the case where contamination such as soot is attached inside the gas pipe 1 or sampling pipe 2, a surface area is increased correspondingly, and therefore a more amount of adsorptive gas such as NH₃ is adsorbed. Accordingly, even in the case where the span gas is injected from the gas injecting mechanism 3, as an amount of contamination is increased, an amount of NH₃ gas to be trapped increases, and therefore it takes time for an indicated value to reach the concentration set for the span gas. That is, the contamination determining part 41 senses the contamination with use of responsiveness of the gas measuring mechanism 21, which is deteriorated by the adsorption of the adsorptive gas to the contamination. According to the contamination determining part 41 configured as described, the contamination is sensed on the basis of the adsorptivity of the NH₃ that is adsorptive gas, and therefore contamination that cannot be easily sensed on the basis of a parameter such as a flow rate or another parameter, and particularly adversely influences adsorptive gas measurement can be quickly sensed. The present embodiment is configured to, in the case where contamination is sensed, for example, automatically clean up the gas pipe 1 or sampling pipe 2 to be able to improve a response speed of the gas measuring mechanism 21 in the NH₃ measurement, and prevent a situation such as situation where inaccurate calibration is performed in a state where the span gas is still adsorbed to contamination, and subsequent measurement is continued in a state of including a large error.

Next, operation and the like of each of the parts at the time of measuring the concentration of the NH₃ gas in the exhaust gas after the end of the calibration are described. In addition, the present embodiment is adapted to be able to, after the end of the calibration, supply NH₃ as injection gas to the gas pipe 1 and the sampling pipe 2 at concentration different from that of the span gas by switching the injection gas source 31 to another source. In doing so, the present embodiment is adapted to set the concentration of the NH₃ as the injection gas to a value such as a value equal to or less than a half in a measurable range of the gas measuring mechanism 21, and even in the case where the exhaust gas-derived NH₃ is further added, prevent a concentration indicated value from being saturated in the gas measuring mechanism 21.

The gas injecting mechanism 3 starts to inject the NH₃ as the injection gas into the gas pipe 1 before the exhaust gas flows into the gas pipe 1, i.e., in a state before the automobile starts the engine. In doing so, the present embodiment is adapted to such that the suction pump 22 provided in the sampling pipe 2 also starts driving and thereby the injection gas flows into the sampling pipe 2 as well. Then, as illustrated in Fig. 3(a), after a saturation amount up to which NH₃ is adsorbed on the inner surfaces of the gas pipe 1 and sampling pipe 2 has been reached, the exhaust gas is introduced. For example, after a predetermined time has passed since the gas injecting mechanism 3 started to inject NH₃, the exhaust gas may be introduced, or in the case where gas concentration measured by the gas measuring mechanism 21 is substantially stabilized at the concentration of the gas injected by the gas injecting mechanism 3, the introduction of the exhaust gas may be started.

The predetermined amount that is an amount of the injection gas injected from the gas injecting mechanism 3 is set to at least the same amount as an amount at which, as illustrated in Fig. 3(b), a total adsorption amount of the adsorbing gas and the injection gas adsorbed on the inner surface of the gas pipe 1 and a total peeling amount of the measuring target gas and the injection gas peeled off from the inner surface of the gas pipe 1 are substantially equilibrated.

A change in gas concentration measured by the gas measuring mechanism 21 during a period from the start to stop of the engine as described is described by comparing a measurement result by a conventional exhaust gas analysis device and a measurement result by the exhaust gas analysis device 100 of the present embodiment with each other.

As illustrated in graphs of Fig. 4, in the case of using the conventional exhaust gas analysis device, an NH₃ concentration indicated value does not indicate an actual concentration value immediately after the start of the engine, and a delay in response occurs due to the adsorption of NH₃ on the inner surfaces of the pipes. After a while, the NH₃ concentration indicated value is stabilized at the actual concentration value; however, even though after the engine stop, the exhaust gas does not flow in, NH₃ peeled off from the inner surfaces is detected, and the concentration indicated value gradually decreases.

On the other hand, according to the exhaust gas analysis device 100 of the present embodiment, the NH₃ gas is started to be flowed as the injection gas at a constant concentration value into the gas pipe 1 and the sampling pipe 2 before the start of measurement, and therefore before the introduction of the exhaust gas, the NH₃ concentration indicated value is brought into a state raised by a predetermined concentration indicated value. Further, the NH₃ concentration indicated value is in a state where the adsorption of NH₃ on the inner surfaces and the peeling off of the NH₃ from the inner surfaces are equilibrated as illustrated in Fig. 3(b), and therefore even if the exhaust gas-derived NH₃ is adsorbed on the inner surfaces, substantially the same amount of NH₃ is peeled off from the inner surfaces instead. Accordingly, the response delay due to the adsorption hardly occurs, and therefore the concentration indicated value outputted by the gas measuring mechanism 21 can reproduce an actual concentration change derived from the exhaust gas in substantially real time.

Next, not the case where a large amount of exhaust gas-derived NH₃ flows in as described above, but the case where NH₃ is outputted only by a minute amount in the case where, for example, the engine is cold started, or in another case is described by comparing a measurement result by the conventional exhaust gas analysis device and that by the exhaust gas analysis device 100 of the present embodiment with each other.

As illustrated in graphs of Fig. 5(a), in the case where a minute amount of NH₃ flows into the exhaust gas analysis device 100 immediately after the start of measurement, in the conventional case, the NH₃ is entirely adsorbed on the inner surfaces of the gas pipe 1 and sampling pipe 2, and therefore the gas measuring mechanism 21 cannot even detect the NH₃. Further, even after a predetermined time has passed since the engine start, phenomena such as outputting a smaller value than actual NH₃ concentration and inconformity in response waveform occur, and then an actual value and a measured value coincide with each other to lead to stabilization. Also, after the engine stop, NH₃ is supposed to be undetected; however, NH₃ adsorbed to the gas pipe 1 and sampling pipe 2 is gradually peeled off, and therefore a concentration indicated value is gradually reduced to make it impossible to reproduce an actual waveform. In other words, the conventional case is disadvantageous in that, in addition to being unable to perform measurement in real time, information on time is missing in a measured value obtained by the measurement.

On the other hand, the present embodiment is adapted to start injecting the injection gas before the engine start to saturate an NH₃ adsorption amount on the inner surfaces, and therefore even in the case where there is a minute amount of exhaust gas-derived NH₃ after the engine start, the NH₃ is not adsorbed on any of the inner surfaces, or even in the case where the NH₃ is adsorbed, substantially the same amount of NH₃ is peeled off from the inner surfaces. Accordingly, as illustrated in Fig. 5(b), even in the case where there is a minute amount of exhaust gas-derived NH₃ immediately after the start of measurement, NH₃ can be accurately detected to measure the concentration of the NH₃. That is, in the conventional case, an erroneous determination that NH₃ is not emitted in the first place tends to be made; however, even a minute amount of NH₃ can be accurately measured in real time without losing information on emission time. For this reason, knowledge that has not been obtained in the past can be obtained from exhaust gas measurement to further contribute to development of the urea SCR system.

Further, with reference to Fig. 6, results of actually measuring a rise time and fall time at the time of measuring NH₃ in the exhaust gas analysis device 100 of the present embodiment are described. In the actual measurement, a response speed in the gas measuring mechanism 21 was evaluated under the conditions that a known amount of NH₃ was introduced from an inlet side of the gas pipe 1 in a step input manner with a predetermined amount of NH₃ as the injection gas being flowed from the gas injecting mechanism 3. As illustrated in Fig. 6(a), measurements were made respectively for the cases of (1) without injecting NH₃ from the gas injecting mechanism 3, introducing NH₃ into the gas pipe 1, (2) keeping injecting 13 ppm NH₃ from the gas injecting mechanism 3 as well as introducing NH₃ into the gas pipe 1, and (3) keeping injecting 23 ppm NH₃ from the gas injecting mechanism 3 as well as introducing NH₃ into the gas pipe 1.

It is clear from an enlarged view of a rising period in Fig. 6(b) and an enlarged view of a falling period in Fig. 6(c), rise and fall times under the experimental conditions (1), (2), and (3) were respectively 26 seconds, 6 seconds, and 2 seconds. That is, it turns out that as an amount of injection gas is increased to bring NH₃ closer to the saturation amount up to which NH₃ is adsorbed on the inner surfaces, the rise and fall times tend to be shorter. Also, it is verified that as with the exhaust gas analysis device of the present embodiment, by measuring NH₃ in the exhaust gas with keeping flowing NH₃ from the gas injecting mechanism 3, NH₃ measurement responsiveness of the gas measuring mechanism 21 can be improved.

Other embodiments are described.

In the above-described embodiment, the exhaust gas analysis device is taken as an example to describe the gas analysis device of the present invention; however, the gas analysis device may be one that measures another gas as the sample gas. Also, as the measuring target gas having adsorptivity, NH₃ is taken as an example; however, another adsorptive gas such as HCl or a hydrocarbon (HC) is also possible. Examples of the hydrocarbon include aromatic hydrocarbons such as toluene, alcohols such as methanol and ethanol, high-boiling HCs, and the like. Further, examples of gas having high adsorptivity include polar gases such as NO₂, SO₂, and H₂O.

The predetermined amount may be set not only to the amount at which the equilibrium state between the adsorption and the peeling off can be retained, but such that a value related to quantity of the injection gas, which is indicated by the gas measuring mechanism, is equal to or less than an allowable difference. Note that the allowable difference is one that, for example, represents a preliminarily allowable error amount with respect to a full scale measurable by some measuring instrument, and is specifically represented by a numerical value such as approximately a few % of the full scale. That is, by making an injection gas-derived concentration indicated value equal to or less than an error of a concentration measured value, which is allowed by the gas measuring mechanism, while keeping the equilibrium state, a sufficiently accurate value can be known with little narrowing a measurement range and even without performing an operation such as subtracting an injection gas-derived concentration value from a value that is obtained in order to know an exhaust gas-derived concentration value.

The contamination determining part is configured to sense the presence or absence of contamination on the basis of the response time that is a value related to the measurement response speed to the absorptive gas; however, another related value such as a change rate at the time of changing from some measured value indicated by the gas measuring mechanism to another measured value may be used. That is, the contamination determining part may be one that makes a determination on the basis of a concentration value measured within a predetermined period of time. In the above-described embodiment, the span gas is injected by the gas injecting mechanism, and concentration to be actually measured is formed in a step shape; however, a measured value may be variously changed in a manner such as a rectangular wave, sine wave, or pulse-shaped manner. For example, in the case where the gas measuring mechanism is adapted to measure a pulse-shaped change, the contamination determining part may determine the presence or absence of contamination on the basis of a period of time necessary for the adsorptive gas to be actually detected by the gas measuring mechanism after the adsorptive gas has been injected by the gas injecting mechanism. Also, a place where the adsorptive injection gas is injected by the gas injecting mechanism is anywhere as long as the place is located in the flow path through which the sample gas flows, and upstream of the measuring point M of the gas measuring mechanism, and for example, the present invention may be adapted to inject the adsorptive injection gas into the sampling pipe. Further, the contamination determining part may be one that not only senses the presence or absence of contamination but determines a level of the contamination on the basis of a measured value.

Further, the contamination determining part may be configured to determine that, in the case where a response time to nonadsorptive gas is obtained, and the response time to the adsorptive gas and the response time to the nonadsorptive gas are substantially the same, maintenance of the suction pump is necessary, or leakage occurs, and in the case where only the response time to the adsorptive gas is longer, contamination is present in the flow path through which the measuring target gas flows. For example, in the case where contamination is present, the response time to the adsorptive gas is deteriorated and increased due to a change in surface area inside the pipes, whereas even in the case where the surface area is changed, the nonadsorptive gas is not significantly influenced, and the response time is little changed. On the other hand, in the case where the suction pump has a trouble, the response times to both of the adsorptive gas and the nonadsorptive gas are longer because it takes longer times for both of the gases to arrive at the gas measuring mechanism. As described, by comparing the response times to the adsorptive and nonadsorptive gases with each other, the contamination determining part can exactly determine causes for two different response delays. In the case of employing such a configuration, the gas measurement device is preferably one that can simultaneously measure multi-component gas, and specific examples of the nonadsorptive gas include CO, CO₂, NO, N₂O, and the like.

Also, the contamination determining part is not one used only for the exhaust gas analysis device but may be one used for another gas analysis device.

In addition, the gas measuring mechanism is one that can measure multi-component gas by the FTIR, but may be one that can measure only another adsorptive gas. Specifically, the gas measuring mechanism may be one that can measure only adsorptive gas such as NH₃ as with NDIR or laser measurement. Also, a value measured by the gas measuring mechanism is not limited to concentration, but may be a value related to quantity of adsorptive gas, such as a flow rate or volume. In addition, the measuring point of the gas measuring mechanism is not in the sampling pipe but may be provided in the gas pipe. In short, it is only necessary that the present invention is adapted such that the injection gas injected from the gas injecting mechanism flows together with the measuring target gas from upstream of the measuring point.

Also, the gas measuring mechanism is placed downstream of the suction pump in the above-described embodiment; however, for example, the gas measuring mechanism may be placed upstream of the suction pump. Even in the case of employing a so-called reduced pressure flow configuration as described, the same effects on contamination sensing and measurement of measuring target gas having adsorptivity as those described above can be obtained.

Besides, it should be appreciated that unless contrary to the scope of the present invention, various combinations and modifications of the embodiments may be made.

### Industrial Applicability

As described, the present invention is configured to sense the presence or absence of contamination with use of a reduction in response speed, which is caused by the adsorption of the adsorptive injection gas on the inner surfaces, and can therefore accurately detect even a small amount of contamination. Accordingly, the flow path can be appropriately cleaned to preferably keep a response speed in adsorptive gas measurement that is influenced even by a small amount of contamination. From these, applying the present invention enables an accurate exhaust gas analysis device to be provided.

## Claims

1. A gas analysis device comprising:
a gas injecting mechanism that injects adsorptive injection gas into a flow path through which sample gas is flowed;
a gas measuring mechanism that can measure a value related to quantity of the adsorptive gas flowing through the flow path; and
a contamination determining part that determines contamination in the flow path on a basis of a value related to a measurement response speed of the gas measuring mechanism to the adsorptive gas.

2. The gas analysis device according to claim 1, wherein:
the flow path is formed in a gas pipe through which the sample gas is flowed, and in a sampling pipe for sampling part of the sample gas; and
the gas measuring mechanism is provided in the sampling pipe, and the contamination determining part is configured to determine contamination on an inner surface of the sampling pipe on a basis of the value related to the measurement response speed.

3. The gas analysis device according to claim 2, wherein
the gas injecting mechanism is configured to inject the adsorptive injection gas into the gas pipe.

4. The gas analysis device according to claim 1, wherein
the contamination determining part is configured to determine that, in a case where a measurement response time necessary for at least a predetermined amount of adsorptive gas to be measured by the gas measuring mechanism after the adsorptive injection gas has been injected by the gas injecting mechanism is equal to or more than a predetermined time, the contamination is present in the flow path.

5. A contamination detection method for a flow path through which sample gas is flowed in a gas analysis device, the contamination detection method comprising:
a gas injecting step of injecting adsorptive injection gas into the flow path;
a gas measuring step of measuring a value related to quantity of the adsorptive gas flowing through the flow path; and
a contamination determining step of determining contamination in the flow path on a basis of a value related to a measurement response speed to the adsorptive gas in the gas measuring step.
